# EUROPEAN PATENT APPLICATION

(11) **EP 2 799 015 A2**
(43) Date of publication of application: **05.11.2014**
(21) Application number: 12861679.4
(22) Date of filing: 27.12.2012
(51) Int. Cl.: A61B 17/04

(54) **ENDOLOOP HAVING A SCREW-COUPLED REAR END PORTION**

(30) Priority: 28.12.2011 KR 20110144111
(71) Applicant: Kim, Jin Sung, Yeonsoo-gu, Incheon 406-756 (KR)
(72) Inventor: Kim, Jin Sung, Yeonsoo-gu, Incheon 406-756 (KR)
(74) Representative: Belloni, Giancarlo
(86) International application number: PCT/KR2012/011652
(87) International publication number: WO 2013/100671

(57) **Abstract**

An endoloop having a screw-coupling rear end section. Screw threads which respectively engage with each other are formed at a portion of a hollow rod and a portion of a rear end section which engage with each other such that the hollow rod and the rear end portion can be coupled with or separated from each other through screw engagement. The rear end section can be separated from the hollow rod even without being applied with strong force. A stitching procedure using the endoloop can be carried out more easily.

## Description

### TECHNICAL FIELD

The present invention relates to an endoloop, and more particularly, to an endoloop having a suture which is used in a surgical procedure using an endoscope so as to be inserted into a human body to stitch a surgical site inside the human body.

### BACKGROUND ART

An endoloop is a surgical instrument which has the shape of an elongated rod and in which a suture is exposed at the leading end thereof. In a surgical procedure using an endoscope, such as laparoscopic surgery, the endoloop is inserted through a small hole and is used to stitch a surgical site while the inside of a human body is being observed with an endoscope.

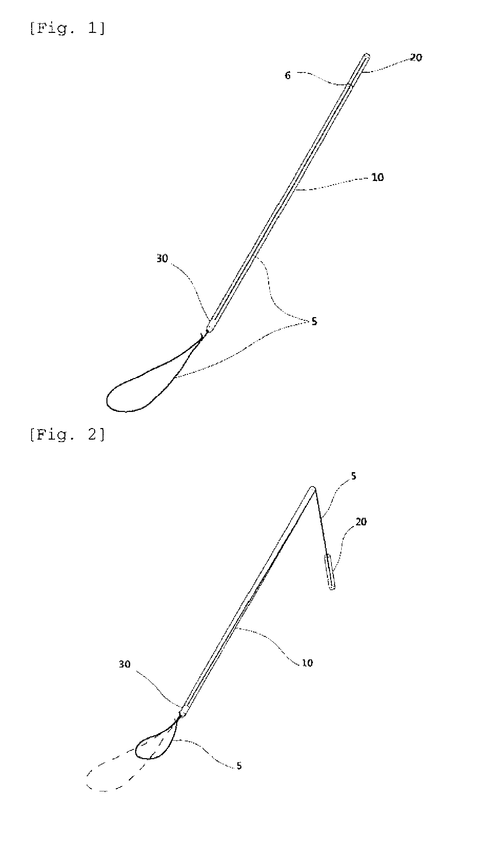

An example of this endoloop that is typically used will be described with reference to FIG. 1 and FIG. 2.

FIG. 1 shows an overall shape of an endoloop that is used in the related art.

A hollow rod 10 that defines the body of the endoloop is shaped as a hollow elongated rod such that a suture 5 can be accommodated therein. As shown in the figures, the suture is also implemented as a transparent plastic material. As shown in the figure, in the state where the suture 5 is accommodated inside the hollow rod 10, one end of the suture 5 is fixed to a rear end section 20 of the hollow rod 10 and the other end of the suture 5 is exposed to the outside through a through-hole formed in the leading end 30.

In the shown example, the suture 5 is prepared such that the other end forms a ring knot.

With this configuration, it is possible to insert the leading end of the endoloop through a small hole that is formed in the skin for the purpose of access to a surgical site inside the human body and stitch the surgical site with the other end of the suture 5.

In order to perform this process, the endoloop of the related art shown in FIG. 1 has a tearing portion 6 at which the rear end section 20 is to be separated from the hollow rod 10. The tearing portion 6 has the shape of a circular groove which is formed at a preset position where the rear end section 20 can be separated.

Specifically, a practitioner locates the leading end 30 at a point to be stitched (hereinafter referred to as the "stitching point") inside the human body, and then separates the rear end section 20 from the hollow rod 10 in a pressure cutting method of applying force to the rear end section 20 to be bent from the hollow rod 10. Afterwards, the practitioner spaces the rear end section 20, to which one end of the suture 5 is fixed, away from the hollow rod 10, as shown in FIG. 2 in which the rear end section 20 is separated, so that the other end of the suture 5 positioned at the surgical site is constricted from the shape indicated by a dotted line into the shape indicated by a solid line. In this fashion, the stitching process is carried out.

The above-described endoloop of the related art has several drawbacks.

First, the hollow rod 10 is made of a polymer resin material that can be broken by force, and the rear end section 20 can be separated from the hollow rod 10 by applying force to the groove-shaped tearing portion 6. When stitching a surgical site that is precisely set with the suture 5, the position of the other end of the suture 5 may deviate from the stitching site while the rear end section 20 is being bent by force applied thereto, thereby making the surgical process difficult. The hollow rod 10 may be broken into pieces at the tearing portion, thereby causing a hygienic problem in the surgery. In particular, a fragment can cause a serious problem when it penetrates into the human body.

Second, since the leading end section 30 has corners which form an angled shape, the leading end section 30 may damage an internal organ while being inserted into the human body.

Third, since the hollow rod 10 is made of a plastic material that has a preset level of softness, the hollow rod 10 tends to be bent while being inserted into the human body. It is sometimes difficult to position the leading end at a suitable surgical position.

### DISCLOSURE

### Technical Problem

The present invention has been made to solve the foregoing problems with the endoloop of the related art, and therefore an object the present invention is to provide an endoloop having a screw-coupling rear end section which is configured such that a rear end section can be easily separated from a hollow rod without being applied with unnecessarily strong force.

Another object of the present invention is to provide an endoloop which lowers the risk of damage to an internal organ while the endoloop is being inserted into the human body.

A further object of the present invention is to provide an endoloop which helps correctly locate a stitching site while the endoloop is being inserted to the stitching site.

Further another object of the present invention is to provide an endoloop in which a fabrication process can be simplified and a fabrication cost can be reduced.

### Technical Solution

In order to realize the object, an aspect of the present invention provides an endoloop that includes an elongated hollow rod and a suture. A space is defined inside the hollow rod. One end of the suture being fixed to a rear end section which is positioned at the rear portion of the hollow rod, and the other end of the suture being exposed to the outside through a through-hole of a leading end section of the hollow rod. One end of the suture is fixed to the inner portion of the rear end section which is separable from the hollow rod. A portion of the hollow rod and a portion of the rear end section which engage with each other are respectively provided with threads which engage with each other, such that the rear end section is to be coupled to and separated from the hollow rod through screw engagement.

Here, the rear end section may have a space defined therein as in the hollow rod. The rear end section may include a suture-fixing device which is fitted into the space of the rear end section and fixedly coupled to the rear end section, and has a suture hole extending the length thereof.

In addition, the leading end section may have a hemispherical surface in which the through-hole is formed.

Here, the leading end section may be configured so as to be separable from the hollow rod. A portion of the hollow rod and a portion of the leading end section which engage with each other are respectively provided with threads which engage with each other, such that the leading end section is to be coupled to and separated from the hollow rod through screw engagement.

In addition, the hollow rod may be made of a hard material such as an aluminum alloy.

### Advantageous Effects

According to the configuration as set forth above, the endoloop having a screw-coupling rear end section according to the invention has the following effects.

First, the rear end section can be separated from the hollow rod without being applied with strong force, thereby allowing a surgical procedure using the endoloop to be more easily carried out.

Second, unlike when the endoloop of the related art is separated, there is no risk of occurrence of fragments when the rear end section is separated. The surgical procedure can be carried out more hygienically.

Third, since the leading end section has a hemispherical shape, it is possible to significantly reduce the risk of damage to an internal organ that would occur while the endoloop is being inserted.

Fourth, since the hollow rod is made of a hard material such as an aluminum alloy, the process of correctly locating the leading end section at a stitching site can be more easily carried out.

Fifth, since the endoloop includes components that can be mass-produced and components that can be easily assembled, the endoloop is easy to fabricate and the fabrication cost can be reduced.

### DESCRIPTION OF DRAWINGS

FIG. 1 is a perspective view showing an endoloop of the related art;
FIG. 2 is a view showing the process of constricting suture by separating a rear end section of the endoloop shown in FIG. 1;
FIG. 3 is a perspective view showing an endoloop according to an exemplary embodiment of the invention;
FIG. 4 is an exploded perspective view showing a rear end section of the endoloop shown in FIG. 3; and
FIG. 5 is an exploded perspective view of a leading end section of the endoloop shown in FIG. 3.

### BEST MODE

Hereinafter the constitution of an endoloop having a screw-coupling rear end section according to the present invention will be described in more detail with reference to the accompanying drawings, in which exemplary embodiments of the present invention are shown.

In the description of the endoloop having a screw-coupling rear end section according to the present invention, the same reference numerals will be used throughout to refer to parts that are the same as or similar to those of the endoloop of the related art in order to avoid unnecessary misunderstanding.

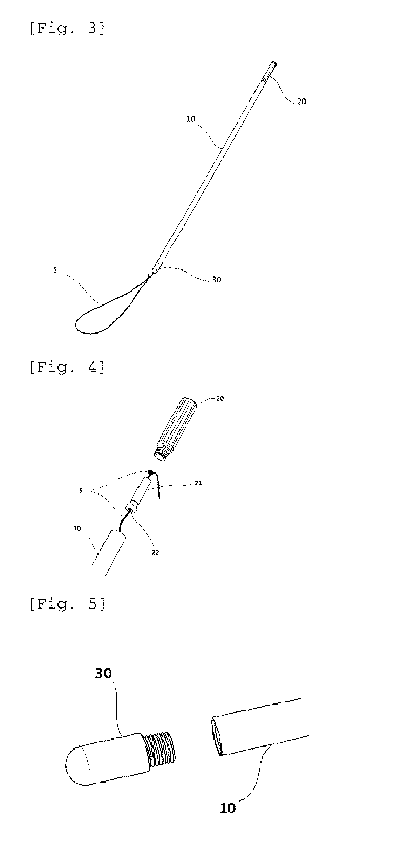

FIG. 3 is a perspective view showing the overall structure of an endoloop having a screw-coupling rear end section according to an exemplary embodiment of the present invention.

As shown in the figures, like the related-art endoloop shown in FIG. 1, the endoloop according to an exemplary embodiment of the present invention also has a hollow rod 10 having the shape of an elongated rod and a suture 5 which is exposed to the outside through a through-hole in a leading end section 30 of the hollow rod 10.

In contrast, unlike the endoloop of the related art, a rear end section 20 of the endoloop according to an exemplary embodiment of the present invention does not have the tearing portion 6 but can be fastened to or separated from the rod through screw engagement.

FIG. 4 is an exploded perspective view showing a rear end section of the endoloop shown in FIG. 3, in which the rear end section 20 is separated from the hollow rod 10.

The rear end section 20 is provided with male threads which form a fastening portion, as shown in the figures, and the hollow rod 10 is provided with female threads in a corresponding portion. With this configuration, the rear end section 20 that is fastened can be easily separated from the hollow rod 10 by the operation of turning the rear end section 20 in the unfastening direction.

In the meantime, an exemplary embodiment of the configuration of fixing the suture 5 to the rear end section 20 is shown in FIG. 4.

As shown in FIG. 4, the rear end section 20 has defined a space therein like the hollow rod 10, and further includes a suture-fixing device 21 which is shaped as a rod that can be inserted into the space of the rear end section 20. The suture-fixing device 21 has a suture hole 22. With this configuration, the process of fixing one end of the suture 5 to the rear end section 20 can be carried out easily.

Specifically, after one end of the suture 5 is hung on the suture-fixing device 21 through the process of forming a knot by passing the suture 5 through the suture hole 22, the suture-fixing device 21 is inserted into the space of the rear end section 20 and fixed to the rear end section 20. Consequently, the suture 5 can be firmly fixed to the rear end section 20.

In order to facilitate the manufacture of a product, both ends of the hollow rod 10 are symmetrically designed. One end of the hollow rod 10 has the female thread that couples with the rear end section 20. The other end of the hollow rod 10 has the same shape, and the leading end section 30 also has a screw-coupling structure like the rear end section 20. Accordingly, mass production of the hollow rod 10 can be maximized.

Advantages involving the screw engagement structure of the leading end section 30 and the hollow rod 10 will be described later in more detail with reference to FIG. 5.

FIG. 5 is an exploded perspective view of the leading end section of the endoloop having a screw-coupling rear end section according to an exemplary embodiment of the present invention.

As shown in the figure, the leading end section 30 of the endoloop according to an exemplary embodiment of the present invention has a hemispherical shape.

Since the leading end section 30 having this shape is provided, the risk of stimulating and damaging an internal organ during insertion of the endoloop into the human body is significantly reduced compared to that of a related-art endoloop having an angled corner, for example, that shown in FIG. 1.

Furthermore, as shown in the figures, the leading end section 30 is configured such that it can be separated from and coupled to the hollow rod 10 through screw engagement. It is therefore possible to facilitate the fabrication process of the endoloop.

Specifically, when the other end of the suture 5 with one end thereof fixed to the rear end section 20 is intended to be exposed in the direction toward the leading end section 30 by passing through the space of the hollow rod 10, there is required a difficult process of passing the other end of the suture 50 through a narrow through-hole if the leading end section 30 is fixed to the corresponding end of the hollow rod 10. In contrast, after the other end of the suture 5 is exposed to the corresponding end of the hollow rod 10 in the state in which the leading end section 30 is separated from the hollow rod 10 as shown in the figures, the process of passing the suture 5 through the through-hole in the leading end section 30 which is relatively short can be easily carry out. After that, the fabrication of the endoloop according to the present invention can be completed merely by fastening the leading end 30 to the hollow rod 10 through screw engagement.

When the screw fastening structure such as the rear end section 20 according to the present invention is applied, it is preferred that the hollow rod 10 or the like be made of a hard material, such as an aluminum alloy or other materials.

When the endoloop is made of a material that has a certain level of softness as in the related art, the hollow rod 10 may be bent while being inserted into the human body. It is therefore difficult to correctly set a stitching site. The hard material has another advantage in that it can overcome this drawback.

Although the exemplary embodiment of the present invention has been described hereinabove in conjunction with the accompanying drawings, it should not be understood that the present invention is limited to the configurations and functions of the embodiment that have been shown and described above. Rather, it will be apparent to persons skilled in the art that a variety of alterations and modifications is possible without departing from the principle and scope of the appended Claims. It should be therefore interpreted that all such alterations and modifications as well as equivalents fall within the scope of the present invention.

**<Description of the Reference Numerals>**

| | | | |
|---|---|---|---|
| 5: | suture | 6: | tearing portion |
| 10: | hollow rod | 20: | rear end section |
| 21: | suture-fixing device | 22: | suture hole |
| 25: | rear end space | 30: | rear end section |

### INDUSTRIAL APPLICABILITY

Since the endoloop according to the invention includes components that can be mass-produced and components that can be easily assembled, the endoloop is easy to fabricate and the fabrication cost can be reduced. Therefore, the endoloop has industrial applicability.

## Claims

1. An endoloop comprising: an elongated hollow rod (10) having a space defined therein; and a suture (5), one end of the suture being fixed to a rear end section (20) which is positioned at a rear portion of the hollow rod (10), and the other end of the suture being exposed to an outside through a through-hole of a leading end section (30) of the hollow rod (10),
wherein one end of the suture (5) is fixed to an inner portion of the rear end section (20) which is separable from the hollow rod (10), and a portion of the hollow rod (10) and a portion of the rear end section (20) which engage with each other are respectively provided with threads which engage with each other, such that the rear end section (20) is to be coupled to and separated from the hollow rod (10) through screw engagement, and
wherein the rear end section (20) has a space defined therein as in the hollow rod, and comprises a suture-fixing device (21) which is fitted into the space of the rear end section (20) and fixedly coupled to the rear end section (20), and has a suture hole (22) extending a length thereof.

2. An endoloop comprising: an elongated hollow rod (10) having a space defined therein; and a suture (5), one end of the suture being fixed to a rear end section (20) which is positioned at a rear portion of the hollow rod (10), and the other end of the suture being exposed to an outside through a through-hole of a leading end section (30) of the hollow rod (10),
wherein one end of the suture (5) is fixed to an inner portion of the rear end section (20) which is separable from the hollow rod (10), and a portion of the hollow rod (10) and a portion of the rear end section (20) which engage with each other are respectively provided with threads which engage with each other, such that the rear end section (20) is to be coupled to and separated from the hollow rod (10) through screw engagement, and wherein the leading end section (30) has a hemispherical surface in which the through-hole is formed.

3. The endoloop according to claim 2, wherein the leading end section (30) is configured so as to be separable from the hollow rod (10), and a portion of the hollow rod (10) and a portion of the leading end section (30) which engage with each other are respectively provided with threads which engage with each other, such that the leading end section (30) is to be coupled to and separated from the hollow rod (10) through screw engagement.

4. The endoloop according to claim 1 or 2, wherein the hollow rod (10) comprises a hard material such as an aluminum alloy.
